# EUROPEAN PATENT SPECIFICATION

(11) **EP 1 258 726 B1**
(45) Date of publication and mention of the grant of the patent: **20.06.2007**
(21) Application number: 01901448.9
(22) Date of filing: 19.01.2001
(51) Int. Cl.: G01N 33/53, G01N 33/76, G01N 33/50

(54) **METHOD OF DETECTING OR QUANTIFYING ENVIRONMENTAL HORMONES**
VERFAHREN ZUM ERKENNEN ODER QUANTIFIZIEREN VON UMWELTHORMONEN
RECHERCHE OU DOSAGE DE PERTURBATEURS ENDOCRINIENS

(30) Priority: 20.01.2000 JP 2000011329
(43) Date of publication of application: 20.11.2002
(73) Proprietor: KYOWA HAKKO KOGYO CO., LTD., Chiyoda-ku, Tokyo 100-8185 (JP)
(72) Inventor: MINEGISHI, Takashi, Maebashi-shi, Gunma 371-0825 (JP); MIYAMOTO, Kaoru, Maebashi-shi, Gunma 371-0822 (JP)
(74) Representative: Harding, Charles Thomas
(86) International application number: PCT/JP2001/000329
(87) International publication number: WO 2001/053823

(56) References cited:
- EP-A- 0 678 577
- WO-A1-93/10460
- US-A- 4 764 502
- YOSHIDA SEIICHI ET AL: "Exposure to diesel exhaust affects the male reproductive system of mice" INTERNATIONAL JOURNAL OF ANDROLOGY, vol. 22, no. 5, October 1999 (1999-10), pages 307-315, XP002324187 ISSN: 0105-6263
- LEFAUCHEUR C ET AL: "[Inhibin and cybernins]" PATHOLOGIE-BIOLOGIE. OCT 1982, vol. 30, no. 8, October 1982 (1982-10), pages 727-736, XP009046557 ISSN: 0369-8114
- YANG K P ET AL: "Characterization of an inhibitor for luteinizing hormone receptor site binding." ENDOCRINOLOGY. JAN 1976, vol. 98, no. 1, January 1976 (1976-01), pages 233-241, XP009046558 ISSN: 0013-7227
- HIRAKAWA TAKASHI ET AL: "Effect of 2,3,7,8-tetrachlorodibenzo-p-dioxin on the expression of luteinizing hormone receptors during cell differentiation in cultured granulosa cells" ARCHIVES OF BIOCHEMISTRY AND BIOPHYSICS, vol. 375, no. 2, 15 March 2000 (2000-03-15), pages 371-376, XP002324190 ISSN: 0003-9861
- SHINICHIRO KAWAI ET AL.: 'Suushu no baiyou saibou wo mochiita naibunpitsu kakuran busshitsu no screening' KANKYOU GIJUTSU vol. 28, no. 5, 1999, pages 300 - 306, XP002955576
- KOUCHI KURODA: 'Kankyou hormone/kankyou estrogen' KANKYOU GIJUTSU vol. 27, no. 12, 1998, pages 895 - 900, XP002955577
- HIDEO KANEKO ET AL.: 'Josei hormone you busshitsu no kenshutsu kei' KAGAKU vol. 68, no. 7, 1998, pages 598 - 605, XP002955578

## Description

### Technical Field

The present invention relates to a method for the detection or determination of environmental hormones, in particular, dioxins in a sample using granulosa cells, etc. In the present invention, the change in the amount of an expressed luteinizing hormone (hereinafter referred to as LH) receptor induced by follicle-stimulating hormone (hereinafter referred to as FSH), etc. in granulosa cells is employed as an indicator for the detection or determination of dioxins.

### Background Art

Recently, environmental hormones which disturb the endocrine system, or endocrine disrupting chemicals, have attracted attention for their effect on the human body [Birnbaum, L. S., Toxicol. Lett., 82-83, 743-750 (1995)]. Among them, dioxins have particularly high toxicity [DeVito, M. J. and Birnbaum, L. S., Toxicology, 102, 115-123 (1995)]. "Dioxins" is the collective name for polychlorodibenzo-p-dioxin (PCDD) and its analogous substance, polychlorodibenzofuran (PCDF). Dioxins are chemical substances secondarily formed in the processes of production of agricultural chemicals, incineration of wastes, paper bleaching, etc. There are 210 kinds of homologues and isomers of dioxins varying in the position and the number of chlorine atoms.

In a method generally employed for the detection of dioxins, dioxins are extracted with an organic solvent such as methanol, purified by silica gel chromatography, followed by reconcentration, and then measured by gas chromatography-mass spectrometry (GC-MS) [Gendai Kagaku (Modern Chemistry), the January issue, 38-43 (1999)].

However, measurement of dioxins by such previous methods is unsatisfactory in detection sensitivity and requires many steps and much time. Even with use of a high resolution gas chromatograph-mass spectrometer recently developed, the concentration step is still indispensable for removing impurities and for raising the dioxin concentration. Further, the final amount of samples for evaluation is limited to tens of microliters.

That is, no simple method has so far been known for the detection of dioxins at low concentrations.

There is no report either on a sensitive detection method which enables the direct analysis of a sample in the form of an aqueous solution. In fact, detection using an aqueous solution requires a large amount of sample; the required amount may vary depending upon the dioxin concentration (regulatory limit for waste water: 0.5-2.5 pg/l) but is several to tens of liters in the case of samples with ordinary dioxin concentrations, because the minimum concentration for determination is of the order of 500 ng/l [Q & A Motto Shiritai Kankyo Hormones To Dioxins, (Q & A More about Environmental Hormones and Dioxins), Environmental Research Institute Inc. (1999)].

Known biological methods for the detection or determination of dioxins include: (1) immunoassays for dioxins using antibodies (Japanese Published Unexamined Patent Applications Nos. 272972/87, 14691/88, 288766/89 and 75841/99, U.S. Pat. No. 4798807, U.S. Pat. No. 5429925, U.S. Pat. No. 5674697 and U.S. Pat. No. 4238472); (2) analysis using aryl hydrocarbon (Ah) receptors which are said to be target proteins of dioxins in the liver, or methods in which Ah receptor activity is enhanced (Japanese Published Unexamined Patent Application No. 58163/88, PCT National Publication No. 501883/95, U.S. Pat. No. 4865972, U.S. Pat. No. 5496703, U.S. Pat. No. 5529899, U.S. Pat. No. 5128244, U.S. Pat. No. 5378822, U.S. Pat. No. 5650283, WO 98/03676 and U.S. Pat. No. 5854010); (3) methods utilizing stress promoters of mammals (PCT National Publication No. 503121/97 and O.S Pat. No. 5811231); and (4) methods in which cyclin kinase in cultured cells is measured (WO 94/17413, WO 97/04316 and U.S. Pat No. 5753519).

However, immunoassays are applicable only to the detection of dioxins of known structure, and the detection methods utilizing Ah receptors, stress promoters and cyclin kinase are detection and measurement systems for the detection of dioxins which induce hepatopathy, dermopathy and cancer, respectively. None of them has been put to practical use yet, nor is a detection system to detect dioxins which affect the endocrine system.

Yoshida et al (1999 - International Journal of Andrology 22.307-315) disclosures that exposure to diesel exhaust induces changes in the Leydig cells of mice and decreases daily sperm production in mice.

In the circumstances, there exists a need for developing a simple method for the detection of low concentrations of environmental hormones such as dioxins which act on the endocrine system affecting reproduction etc.

### Disclosure of the Invention

An object of the present invention is to provide a simple and sensitive method for the detection or determination of environmental hormones, in particular, dioxins in a sample.

To solve the above problems, the present inventors made intensive studies, and as a result, they have discovered a simple and sensitive method for the detection or determination of environmental hormones in a sample.

The present invention relates to the following (1) to (9) .
(1) An in vitro method for the detection or determination of an environmental hormone in a sample, which comprises bringing animal cells into contact with the sample, and measuring the change in the amount of a luteinizing hormone (hereinafter referred to as LH) receptor expressed in the animal cells.
(2) The method according to the above (1), wherein the animal cells are selected from the group consisting of granulosa cells, theca cells and Leydig cells.
(3) The method according to the above (1) or (2), wherein the animal cells are derived from an animal selected from the group consisting of humans, monkeys, rats, mice, guinea pigs and rabbits.
(4) The method according to any of the above (1) to (3), wherein the amount of the LH receptor expressed is evaluated by the amount of mRNA for the LH receptor formed in the animal cells.
(5) The method according to any of the above (1) to (4), wherein the LH receptor is an LH receptor which is expressed upon induction by a substance selected from the group consisting of follicle-stimulating hormone (hereinafter referred to as FSH), insulin-like growth factor I (hereinafter referred to as IGF-I) and cyclic AMP (hereinafter referred to as cAMP).
(6) The method according to any of the above (1) to (5), wherein the environmental hormone is selected from the group consisting of dioxins and polychlorinated biphenyl (hereinafter referred to as PCB).
(7) The method according to the above (6), wherein the dioxins are 2,3,7,8-tetrachlorodibenzo-p-dioxin.
(8) The method according to any of the above (1) to (7), wherein the sample is selected from the group consisting of foods and drinks, agricultural chemicals, incinerated wastes and PCB products.
(9) An in vitro method for the detection or determination of an environmental hormone in a sample which is suspected to contain the environmental hormone, which comprises comparing the amount of an LH receptor expressed in a system in which only a substance selected from the group consisting of FSH, IGF-I and cAMP is caused to act on granulosa cells with that in a system in which the sample and a substance selected from the group consisting of FSH, IGF-I and cAMP are caused to act on granulosa cells.

There is mentioned herein a method for contraception which comprises inhibiting the expression of a luteinizing hormone receptor induced by follicle-stimulating hormone in granulosa cells.

There is also mentioned herein a contraceptive agent comprising, as an active ingredient, a substance which inhibits the expression of a luteinizing hormone receptor.

The present invention is described in detail below.

Measurement of environmental hormones according to the present invention comprises bringing animal cells in vitro into contact with a sample and measuring the change in the amount of LH receptors expressed in the animal cells.

The method of the present invention can be used for detecting or determining any environmental hormones that affect the amount of LH receptors to be expressed. These environmental hormones include dioxins such as 2,3,7,8-tetrachlorodibenzo-p-dioxin, and polychlorinated biphenyl (PCB).

Any samples that are suspected to contain the above-described environmental hormones may be assayed by the method of the present invention. Examples of these samples include foods and drinks, agricultural chemicals, incinerated wastes and PCB products.

The samples suspected to contain the environmental hormones may be used in the method of the present invention as such, or after being dissolved, dispersed or emulsified in media such as lower alcohols (e.g. methanol, ethanol and propanol), dimethyl sulfoxide and dimethylformamide. Emulsification can be carried out by a known method.

As the animal cells, any of those capable of expressing LH receptors may be employed. Suitable animal cells include granulosa cells, theca cells and Leydig cells. Preferred are vertebrate-derived granulosa cells. Examples of the vertebrates are humans, monkeys, rats, mice, guinea pigs and rabbits. Particularly preferred are rats.

Preparation of granulosa cells can be carried out by the method of Nakamura, et al. [Nakamura, M., et al., Endocrinology, 133, 538-544 (1993)]. Culturing of granulosa cells can also be carried out by the method of Nakamura, et al. For example, an ovary is taken from an animal and granulosa cells are obtained by making a hole in the ovarian follicle. The obtained granulosa cells are cultured on a plate using a medium for animal cells, for example, Ham's F-12/DME (Gibco Laboratories) in an atmosphere (5% CO₂, 95% air) at around 37°C for over 40 hours.

In order to cause a sample suspected to contain environmental hormones to act on animal cells, the sample is added to a culture of the animal cells and the resulting mixture is cultured by the above-described method. The sample may be added either before or in the course of the culturing.

The expression of LH receptors is induced by follicle-stimulating hormone (FSH), insulin-like growth factor I (hereinafter referred to as IGF-I), cyclic AMP (hereinafter referred to as cAMP), etc. Therefore, the detection or determination of environmental hormones is carried out by comparing the amount of the LH receptors expressed when an inducing substance alone is caused to act on animal cells with that when the inducing substance and environmental hormones are caused to act on animal cells. As the expression of LH receptors is usually inhibited in proportion to the amount of environmental hormones existing, the detection or determination of environmental hormones can be carried out by measuring a decrease in the amount of the LH receptors expressed.

Measurement of the amount of the LH receptors expressed can be carried out by using cells wherein a marker gene such as a luciferase gene is incorporated into the LH receptor gene and measuring the amount of the marker, for example, the luciferase luminescence intensity as the intensity of the transcription activity.

The measurement can also be carried out by measuring the amount of mRNAs encoding the LH receptors formed in the cells, which method is simple and preferable.

The methods for measuring the amount of mRNA include but are not limited to methods using fluorescence and radioactivity [Maniatis, Molecular Cloning, Cold Spring Harbor Laboratory Press (1989)]. Any other methods capable of measuring mRNA either directly or indirectly can also be employed. A specific example is the method for measurement of the amount of mRNA described in Examples of the present invention. It is possible to detect or determine environmental hormones to the level of 1 pmol/l by the method comprising the determination of the amount of the expressed LH receptors by measuring the amount of mRNAs.

There is also described herein a method for contraception comprising inhibiting the expression of LH receptors induced by FSH in granulosa cells. Inhibition of the expression of LH receptors prevents granulosa cells from responding to LH secreted by the pituitary gland, which blocks ovulation. That is, this method is capable of inhibiting ovulation and thus can be employed for contraception, etc.

Inhibition of the expression of LH receptors may be carried out by any method, for example, by administration of a substance having the activity to inhibit the expression of LH receptors (hereinafter referred to as an LH receptor expression inhibitor). Any of the substances that inhibit the expression of LH receptors, for example, environmental hormones may be employed as the LH receptor expression inhibitor. Useful environmental hormones include the above-described environmental hormones, but are preferably those which inhibit the expression of LH receptors and which have lower toxicity to the human body.

The contraceptive agent comprising an LH receptor expression inhibitor as an active ingredient is described below.

The contraceptive agent mentioned herein can be produced according to any of the methods well known in the technical field of pharmaceutics by mixing the above-described LH receptor expression inhibitor, and if necessary additional active ingredients, with one or more pharmaceutically acceptable carriers.

It is desirable to administer the contraceptive agent disclosed herein by a route which is effective for the purpose, for example, oral administration or non-oral administration such as intravenous administration.

The contraceptive agent is administrated in the form of tablets, capsules, injection, drip infusion, syrup, sublingual tablets, various kinds of cream, suppository, or the like.

Liquid preparations suitable for oral administration such as syrup can be produced using water, sugars (e.g., sucrose, sorbitol and fructose), glycols (e.g., polyethylene glycol and propylene glycol), oils (e.g., sesame oil, olive oil and soybean oil), antiseptics (e.g., p-hydroxybenzoates), flavors (e.g., strawberry flavor and peppermint), and the like. Tablets, powders, granules, etc. can be produced using excipients (e.g., lactose, glucose, sucrose and mannitol), disintegrators (e.g., starch and sodium alginate), lubricants (e.g., magnesium stearate and talc), binders (e.g., polyvinyl alcohol, hydroxypropyl cellulose and gelatin), surfactants (e.g., fatty acid esters), plasticizers (e.g., glycerin), and the like.

Preparations suitable for non-oral administration preferably comprise a sterilized aqueous preparation containing an active compound which is isotonic to the recipient's blood. In the case of injection, for example, a solution for injection is prepared using a carrier comprising a salt solution, a glucose solution, or a mixture of salt water and a glucose solution.

The non-oral preparations may also comprise one or more auxiliary components selected from the diluents, antiseptics, flavors, excipients, disintegrators, lubricants, binders, surfactants, plasticizers, etc. mentioned in the above description of oral preparations.

The content of the LH receptor expression inhibitor in the contraceptive agent described herein is 1 to 1000 mg/g, preferably 10 to 500 mg/g, most preferably 100 to 200 mg/g.

The dose and the administration schedule of the contraceptive agent described herein varies depending upon the administration route, the patient's age and body weight, etc.

In the case of oral administration, it is suitable to administer the LH receptor expression inhibitor in an amount of 0.1 to 100 mg, preferably ca. 0.1 to 10 mg per person usually once to several times per day.

In the case of non-oral administration such as intravenous administration, it is suitable to administer the LH receptor expression inhibitor in an amount of 0.01 to 10 mg, preferably ca. 0.01 to 1 mg per adult once to several times per day.

Administration of the contraceptive agent mentioned herein can inhibit the expression of LH receptors, thus achieving the contraceptive effect.

The contraceptive agent disclosed herein: can be used not only for humans, but also for animals other than humans. The dose to animals is the same as that to humans. The expression of LH receptors is measured in the same manner as described above.

The method for the detection of environmental hormones according to the present invention is illustrated in the following examples.

### Brief Description of the Drawings

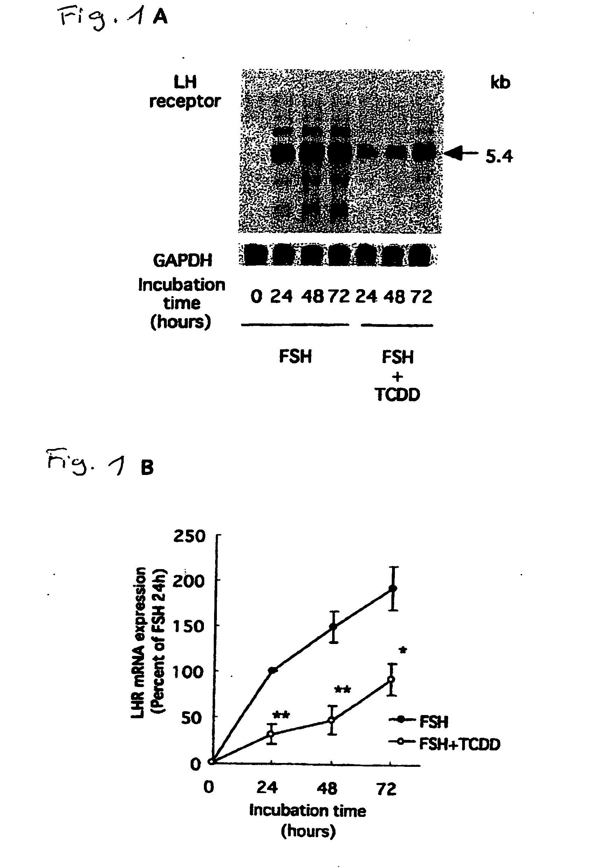

Fig. 1 (A) shows the mode of expression of LH-R mRNA (5.4 kb) and GAPDH mRNA when granulosa cells obtained from a DES (diethylstilbestrol)-treated juvenile rat were cultured for 24 hours and then were allowed to stand in the presence of FSH (30 ng/ml), and FSH (30 ng/ml) plus TCDD (10 pmol/l), respectively, for 24, 48 and 72 hours. Fig. 1 (B) shows the results in (A) quantified by using a densitometer.

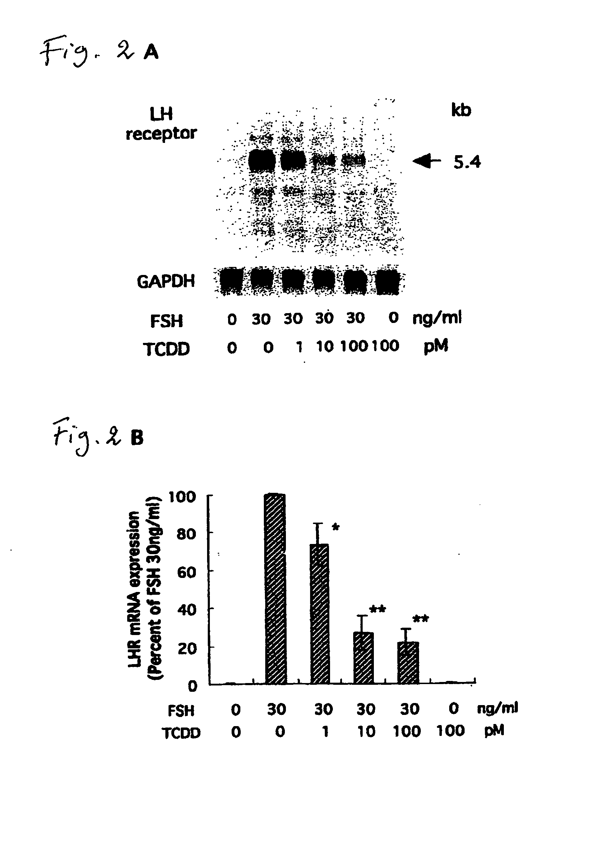

Fig. 2 (A) shows the mode of expression of LH-R mRNA (5.4 kb) and GAPDH mRNA when granulosa cells obtained from a DES-treated juvenile female rat were cultured for 24 hours and then were cultured in the presence of FSH (30 ng/ml) plus various concentrations of TCDD (1, 10, 100 pmol/l) for 48 hours. Fig. 2 (B) shows the results in (A) quantified by using a densitometer.

### Best Modes for Carrying Out the Invention

### Reference Example 1 Preparation of Rat Granulosa Cells

Rat granulosa cells were obtained from a juvenile female Wistar rat (purchased from Charles River Laboratories, Inc.) which had been injected with 2 mg of diethylstilbestrol (produced by Sigma Chemical Co.) dissolved in 0.1 ml of sesame oil once per day for 4 days. Specifically, the ovary of the juvenile female Wistar rat was removed and a hole was made in its follicle with a 25-gauge needle to take out granulosa cells. During this series of operations, the rat was treated according to the NIH guidelines (Biosafety in microbiological and biomedical laboratories). Then, the obtained granulosa cells were washed and centrifuged to collect them. The viability of the cells was determined by the known trypan blue exclusion method.

The obtained granulosa cells (viability: over 90%) were cultured in Ham's F-12/DME (1:1 vol/vol) (produced by Gibco Laboratories) containing 1.1 g/l NaHCO₃, 40 mg/l gentamicin sulfate (produced by Sigma Chemical Co.), 1 mg/l Fungizone (produced by Gibco Laboratories) and 100 mg/l bovine serum albumin (BSA) on a collagen-coated plate in a moist atmosphere (5% CO₂, 95% air) at 37°C [Nakamura, M., et al., Endocrinology, 133, 538-544 (1993)]. The granulosa cells obtained by the culturing were used in Examples.

### Example 1 Microassay for Dioxins Using the Expression of LH Receptors (LH-R) as an Indicator

### (1) Isolation and Analysis of an RNA Mixture

The rat granulosa cells obtained in Reference Example 1 (5 x 10⁶ living cells) were cultured in 5 ml of a serum-free medium [Ham's F-12/DME (1:1 vol/vol)] on a plate of 60 mm diameter for 24 hours, and the resulting culture was allowed to stand separately in the presence of 30 ng/ml rat FSH (I-8) [obtained from National Hormone and Pituitary Distribution Program (Bethesda MD)] and in the presence of FSH (30 ng/ml) and 2,3,7,8-tetrachlorodibenzo-p-dioxin (TCDD) [Sigma Chemical Co.] (1, 10 and 100 pmol/l). TCDD was used after being dissolved in dimethyl sulfoxide.

After culturing was stopped 24, 48 and 72 hours later, RNAs formed in the cells were extracted to obtain pellets of the RNA mixture according to the AGTC method [trade name: Isogen, produced by Nippon Gene Co., Ltd., Chomezynski, P. and Sacchi, N., Anal. Biochem., 162, 156-159 (1987)]. To inhibit RNase, 15 µg of the obtained pellets was dissolved in diethyl-pyrocarbonate-treated water. The resulting solution was subjected to denatured agarose gel electrophoresis and the RNAs were transferred to a nylon membrane (trade name: Biodyne, produced by ICN) to carry out Northern blotting analysis. A film (trade name: Kodak X-Omat, produced by Eastman Kodak Co.) was exposed to the nylon membrane according to the standard manual of a nucleic acid detection kit (produced by Boehringer Mannheim) for fluorescence detection using 2202 UnitroScan Laser Densitometer (produced by LKB Co., Ltd.) to determine the mRNAs.

### (2) Preparation of Rat LH-R cRNA Probe

Rat LH-R mRNA was prepared according to the description in Mol. Cell. Endocrinol., 82, 259-263 (1991). The obtained rat LH-R mRNA was inserted into the EcoRI site of Bluescript ks (+) vector, followed by subcloning and linearization with BglII [Nakamura, K., Nakamura, M., Igarashi, S., Miyamoto, K., Eto, Y., Ibuki, Y., and Minegishi, T., Endocrinology, 134, 2329-2335 (1994)]. Then, digoxigenin-labeled LH-R cRNA corresponding to nucleotide numbers 440 to 2560 of the rat LH-R mRNA was prepared by in vitro transcription using T3 RNA polymerase and an RNA-labeling kit.

### (3) Determination of Rat LH-R mRNA

Each of the rat LH-R mRNAs (5.4 kb) obtained in the above (1) was determined according to the luminescence intensity method. The luminescence intensity was measured with LKB 2202 UnitroScan Laser Densitometer (produced by LKB Co., Ltd.) using the probe prepared in the above (2).

The amount of each rat LH-R mRNA expressed was determined using the amount of GAPDH (glyceraldehyde 3-phosphate dehydrogenase) mRNA expressed as a reference value to obtain the relative value (%) based on the control [addition of only FSH (30 ng/ml)]. The data were expressed in terms of SE which is the average of the values obtained by carrying out culturing three times under the same conditions. Comparison of the values under various conditions was carried out by one-way ANOVA. The significance of difference between the average value of the control and that obtained under each of various conditions was judged by Duncan's multiple comparison test. The difference was statistically significant (P<0.05).

### (4) Detection of Dioxins Using Granulosa Cells

The results of Northern blotting under various conditions with TCDD concentrations of 0 and 10 pmol/l in the above (1) are shown in Fig. 1 (A). A difference in the amount of expressed LH-R mRNA already appeared after 24 hours of standing. It indicates that detection of 10 pmol/l TCDD is possible at least in 24 hours in this detection system.

The results in Fig. 1 (A) quantified by using a densitometer (LKB 2202 UnitroScan Laser Densitometer, produced by LKB Produkter Co., Ltd.) are shown in Fig. 1 (B). All data were determined using the amount of GAPDH mRNA as a reference value regarding the amount of LH-R mRNA expressed by culturing in the presence of FSH (30 ng/ml) for 24 hours as 100% and were expressed as the relative values (N=3) based on the control. In any of the measurements after 24, 48 and 72 hours, the addition of 10 pmol/l TCDD decreased the amount of LH-R mRNA expressed to less than 50% of that without TCDD addition.

### Example 2 Concentration Dependency in Microassay for Dioxins Using the LH-R Expression as an Indicator

The results of Northern blotting after 48 hours of standing under various conditions in the above Example 1 (1) are shown in Fig. 2 (A). The results shown include those obtained without addition of FSH and TCDD, with addition of FSH (30 ng/ml) alone, and with addition of TCDD (100 pmol/l) alone. There was observed a little decrease in the expression of LH-R mRNA by addition of 1 pmol/l TCDD and the expression of LH-R mRNA was clearly inhibited by addition of 10 pmol/l TCDD.

These results indicate that the detection of 1 pmol/l TCDD is possible and the detection of 10 pmol/l TCDD can be satisfactorily carried out by the above method. TCDD is considered to exist in the environment usually at concentrations around 10 pmol/l, and thus this detection system has sensitivity that will be sufficient to analyze samples from the environment.

The results in Fig. 2 (A) quntified by using a densitometer (LKB 2202 UnitroScan Laser Densitometer, produced by LKB Produkter Co., Ltd.) are shown in Fig. 2 (B).

All data were determined using the amount of GAPDH mRNA as a reference value regarding the amount of LH-R mRNA expressed by culturing in the presence of FSH (30 ng/ml) alone for 24 hours as 100% and were expressed as the relative values (N=3) based on the control. The expression of LH-R mRNA was decreased in a manner dependent upon the amount of TCDD added. This tendency indicates that it is possible to determine the amount of TCDD contained in a sample.

### Industrial Applicability

The present invention provides a method for the detection or determination of environmental hormones in a sample.

In accordance with the invention, environmental hormones, in particular, dioxins can be simply and sensitively detected or determined by using granulosa cells and utilizing the expression level of LH receptors as an indicator.

## Claims

1. An in vitro method for the detection or determination of an environmental hormone in a sample, which comprises bringing animal cells into contact with the sample, and measuring the change in the amount of a luteinizing hormone receptor expressed in the animal cells.

2. The method according to Claim 1, wherein the animal cells are selected from the group consisting of granulosa cells, theca cells and Leydig cells.

3. The method according to Claim 1 or 2, wherein the animal cells are derived from an animal selected from the group consisting of humans, monkeys, rats, mice, guinea pigs and rabbits.

4. The method according to any of Claims 1 to 3, wherein the amount of the luteinizing hormone receptor expressed is evaluated by the amount of mRNA for the luteinizing hormone receptor formed in the animal cells.

5. The method according to any of Claims 1 to 4, wherein the luteinizing hormone receptor is a luteinizing hormone receptor which is expressed upon induction by a substance selected from the group consisting of follicle-stimulating hormone, insulin-like growth factor I and cyclic AMP.

6. The method according to any of Claims 1 to 5, wherein the environmental hormone is selected from the group consisting of dioxins and polychlorinated biphenyl.

7. The method according to Claim 6, wherein the dioxins are 2,3,7,8-tetrachlorodibenzo-p-dioxin.

8. The method according to any of Claims 1 to 7, wherein the sample is selected from the group consisting of foods and drinks, agricultural chemicals, incinerated wastes and polychlorinated biphenyl products.

9. An in vitro method for the detection or determination of an environmental hormone in a sample which is suspected to contain the environmental hormone, which comprises comparing the amount of a luteinizing hormone receptor expressed in a system in which only a substance selected from the group consisting of follicle-stimulating hormone, insulin-like growth factor I and cyclic AMP is caused to act on granulosa cells with that in a system in which the sample and a substance selected from the group consisting of follicle-stimulating hormone, insulin-like growth factor I and cyclic AMP are caused to act on granulosa cells.

## Patentansprüche

1. In vitro-Verfahren für die Detektion oder Bestimmung eines Umwelthormons in einer Probe, welches umfaßt, daß man Tierzellen mit der Probe in Kontakt bringt und die Veränderung in der Menge eines in den Tierzellen exprimierten Luteinisierungshormonrezeptors mißt.

2. Verfahren nach Anspruch 1, wobei die Tierzellen aus der Gruppe ausgewählt sind, bestehend aus Granulosazellen, Thecazellen und Leydig-Zellen.

3. Verfahren nach Anspruch 1 oder 2, wobei die Tierzellen von einem Tier abgeleitet sind, ausgewählt aus der Gruppe, bestehend aus Menschen, Affen, Ratten, Mäusen, Meerschweinchen und Kaninchen.

4. Verfahren nach einem der Ansprüche 1 bis 3, wobei die Menge an exprimiertem Luteinisierungshormonrezeptor anhand der Menge an mRNA für den in den Tierzellen gebildeten Luteinisierungshormonrezeptor beurteilt wird.

5. Verfahren nach einem der Ansprüche 1 bis 4, wobei der Luteinisierungshormonrezeptor ein Luteinisierungshormonrezeptor ist, der bei Induzierung durch eine Substanz, ausgewählt aus der Gruppe, bestehend aus Follikel-stimulierendem Hormon, Insulin-artigem Wachstumsfaktor I und zyklischem AMP, exprimiert wird.

6. Verfahren nach einem der Ansprüche 1 bis 5, wobei das Umwelthormon aus der Gruppe ausgewählt ist, bestehend aus Dioxinen und polychloriertem Biphenyl.

7. Verfahren nach Anspruch 6, wobei die Dioxine 2,3,7,8-Tetrachlordibenzo-p-dioxin sind.

8. Verfahren nach einem der Ansprüche 1 bis 7, wobei die Probe aus der Gruppe ausgewählt ist, bestehend aus Nahrungsmitteln und Getränken, landwirtschaftlichen Chemikalien, verbrannten Abfällen und polychlorierten Biphenylprodukten.

9. In vitro-Verfahren für die Detektion oder Bestimmung eines Umwelthormons in einer Probe, von welcher angenommen wird, daß sie das Umwelthormon enthält, wobei das Verfahren umfaßt, daß man die Menge eines Luteinisierungshormonrezeptors, der in einem System exprimiert wird, in dem nur eine Substanz, ausgewählt aus der Gruppe, bestehend aus Follikel-stimulierendem Hormon, Insulin-artigem Wachstumsfaktor I und zyklischem AMP, dazu gebracht wird, auf Granulosazellen zu wirken, mit der Menge in einem System vergleicht, in dem die Probe und eine Substanz, ausgewählt aus der Gruppe, bestehend aus Follikel-stimulierendem Hormon, Insulin-artigem Wachstumsfaktor I und zyklischem AMP, dazu gebracht werden, auf Granulosazellen zu wirken.

## Revendications

1. Méthode in vitro pour la détection ou la détermination d'une hormone ambiante dans un échantillon, qui comprend les étapes consistant à mettre des cellules animales en contact avec l'échantillon, et à mesurer la variation de la quantité d'un récepteur d'hormone lutéinisante exprimé dans les cellules animales.

2. Méthode suivant la revendication 1, dans laquelle les cellules animales sont choisies dans le groupe consistant en des cellules de la granulosa, des cellules de la thèque et des cellules de Leydig.

3. Méthode suivant la revendication 1 ou 2, dans laquelle les cellules animales sont dérivées d'un animal choisi dans le groupe consistant en les êtres humains, les singes, les rats, les souris, les cobayes et les lapins.

4. Méthode suivant l'une quelconque des revendications 1 à 3, dans laquelle la quantité de récepteur d'hormone lutéinisante exprimé est évaluée par la quantité d'ARNm pour le récepteur d'hormone lutéinisante formé dans les cellules animales.

5. Méthode suivant l'une quelconque des revendications 1 à 4, dans laquelle le récepteur d'hormone lutéinisante est un récepteur d'hormone lutéinisante qui est exprimé par induction par une substance choisie dans le groupe consistant en la folliculostimuline, le facteur de croissance I du type insuline et l'AMP cyclique.

6. Méthode suivant l'une quelconque des revendications 1 à 5, dans laquelle l'hormone environnementale est choisie dans le groupe consistant en des dioxines et le biphényle polychloré.

7. Méthode suivant la revendication 6, dans laquelle les dioxines consistent en la 2,3,7,8-tétrachlorodibenzo-p-dioxine.

8. Méthode suivant l'une quelconque des revendications 1 à 7, dans laquelle l'échantillon est choisi dans le groupe consistant en des aliments et des boissons, des agents agrochimiques, des résidus d'incinération et des biphényles polychlorés.

9. Méthode in vitro pour la détection ou la détermination d'une hormone ambiante dans un échantillon qui est supposé contenir l'hormone ambiante, qui comprend la comparaison de la quantité d'un récepteur d'hormone lutéinisante exprimé dans un système dans lequel seule une substance choisie dans le groupe consistant en la folliculostimuline, le facteur de croissance I du type insuline et l'AMP cyclique est amenée à agir sur des cellules de la granulosa avec celle dans un système dans lequel l'échantillon et une substance choisie dans le groupe consistant en la folliculostimuline, le facteur de croissance I du type insuline et l'AMP cyclique sont amenés à agir sur des cellules de la granulosa.
